# EUROPÄISCHE PATENTSCHRIFT

(11) **EP 2 734 145 B1**
(45) Veröffentlichungstag und Bekanntmachung des Hinweises auf die Patenterteilung: **12.06.2019**
(21) Anmeldenummer: 12737778.6
(22) Anmeldetag: 17.07.2012
(51) Int. Cl.: A61C 8/00

(54) **AUFBAUTEIL FÜR EINEN KÜNSTLICHEN ZAHNERSATZ**
ABUTMENT FOR AN ARTIFICIAL DENTAL PROSTHESIS
PILIER POUR UNE PROTHESE DENTAIRE ARTIFICIELE

(30) Priorität: 19.07.2011 DE 102011051930; 12.08.2011 DE 102011052644
(43) Veröffentlichungstag der Anmeldung: 28.05.2014
(73) Patentinhaber: ZV3 - Zircon Vision GmbH, 82515 Wolfratshausen (DE)
(72) Erfinder: FEITH, Johan, 82547 Eurasburg (DE)
(74) Vertreter: Bohnenberger, Johannes
(86) Internationale Anmeldenummer: PCT/EP2012/063939
(87) Internationale Veröffentlichungsnummer: WO 2013/011003

(56) Entgegenhaltungen:
- EP-A1- 1 319 375
- DE-A1-102005 013 200
- DE-A1-102006 045 186
- DE-A1-102006 059 515
- DE-A1-102007 026 325
- US-A- 5 816 809
- US-A1- 2003 054 318
- US-A1- 2003 082 499
- US-A1- 2003 228 556

## Beschreibung

Die vorliegende Erfindung betrifft ein Aufbauteil für einen künstlichen Zahnersatz, einen künstlichen Zahnersatz mit einem entsprechenden Aufbauteil sowie ein Verfahren zur Herstellung und/oder Implantierung eines künstlichen Zahnersatzes.

Aus dem Stand der Technik (DE 10 2006 045 186 A1) sind Aufbauteile für ein zweiteiliges Zahnimplantat bekannt.

Die DE 10 2006 059 515 A1 schlägt vor, den in den Knochen einzubringenden Teil eines Dentalimplantats mittels eines geeigneten Werkzeugs, welches von seiner geometrischen Form her zum Implantat passt um ein Drehmoment zu übertragen, in den Knochen zu schrauben.

Die US 5,816,809 A zeigt ein Verbindungsstück zwischen einem in den Knochen implantierbaren distalen Stück und einer darauf aufzusetzenden Krone, wobei das Verbindungsstück aus Titan, Stahl oder faserverstärkte Harzkomposite hergestellt wird und das obere Ende des Verbindungsstücks eine Sollbruchstelle aufweist.

Des Weiteren sind zweiteilige Dentalimplantate aus der US 2003/0104338 A1 bekannt, die sich aus einem Verankerungsteil und einem Aufbauteil oder Abutment zusammensetzen. Nach der Implantation des Verankerungsteils und dem Abwarten eines möglicherweise notwendigen Einheilzeitraums wird auf das Verankerungsteil das Aufbauteil aufgeschraubt oder aufgeklebt. Das Aufbauteil trägt dann die Krone oder die entsprechende Suprakonstruktion. Zwischen dem Verankerungsteil und dem Aufbauteil besteht ein Spalt, in welchem sich Bakterien ansiedeln können. Dadurch kann es mitunter zu einer bakteriell bedingten Knochenrückbildung kommen. Aufgrund der Knochenrückbildung ändert sich auch der Verlauf der natürlichen Zahnfleischgrenze. Das Zahnfleisch fällt ein bzw. verlagert sich so, dass mitunter Übergänge zwischen Implantat und Krone sichtbar werden. Dies stört den optischen Eindruck des künstlichen Zahnersatzes. Besonders störend und ästhetisch unerwünscht sind freiliegende Implantate aus Titan.

Die DE 101 59 683 A1 schlägt daher vor, einteilige Implantate, insbesondere auf Zirkonoxidbasis zu verwenden, wobei Aufbauteil und Verankerungsteil aus einem Stück gefertigt sind. Derartige einteilige Implantate sind nach dem Sintern mit herkömmlichen Mitteln praktisch nicht beschleifbar. Das heißt, das Zirkonoxid lässt sich zwar auch im gesinterten Zustand beschleifen, wobei jedoch Mikrorisse auftreten, wodurch der künstliche Zahnersatz mitunter unbrauchbar wird.

Des Weiteren wird beim Beschleifen von Zirkonoxid das Material derart erhitzt, dass an dem Implantat anliegende Zellen absterben. Ein nachträgliches Anpassen des Implantats ist daher nicht möglich. Entsprechend hoch sind die Anforderungen an den Implantatsherstellungsprozess sowie an die Person, die das Implantat setzt.

Die US 60/438,266 schlägt ein zweiteiliges Implantat, umfassend ein Verankerungsteil und ein Aufbauteil aus Titan, vor. Das Aufbauteil ist teilweise in das Verankerungsteil eingesteckt und mit diesem über ein Gewinde verbunden. Das Implantat hat eine Außenkontaktfuge, die beim gesetzten Implantat im Knochen deutlich beabstandet ist. Das Implantat ist derart ausgebildet, dass die Außenkontaktfuge zumindest abschnittsweise von einer anzubringenden Krone verschlossen wird.

Auch Titan-Implantate sollten *in situ* nicht beschliffen werden. Da derartige Implantate ebenfalls über eine hohe Wärmeleitfähigkeit verfügen, verteilt sich die lokal, durch das Abschleifen bedingte Erwärmung, über das gesamte Implantat. Dieses erwärmt sich, und Knochenzellen, die in direktem Kontakt mit dem Implantat stehen, sterben ab. Ein eventuell bereits erzielter Einheilerfolg wird umgekehrt. Des Weiteren können Metallsplitter, die beim Abschleifen abgelöst und durch das Schleifwerkzeug stark beschleunigt werden, in das Zahnfleisch des Patienten eindringen. Ein nachträgliches Herauslösen dieser Splitter ist häufig nicht möglich. Sie verbleiben im Zahnfleisch und färben dieses mitunter optisch auffällig ein.

Unter Berücksichtigung dieser Unzulänglichkeiten schlägt die EP 2 146 665 vor, ein dreiteiliges Zahnimplantat bestehend aus einem Verankerungsteil, einem Aufbauteil und einer Krone zu verwenden. Das Aufbauteil soll vorzugsweise aus Kunststoff gefertigt werden, so dass sich dieses einfach beschleifen lässt. Das Verankerungsteil soll zumindest abschnittsweise aus einer technischen Keramik gefertigt werden, die eine deutlich höhere Härte aufweist als der Kunststoff des Aufbauteils. Bei der Verwendung von technischen Keramiken stellt sich das Problem, dass diese mit hoher Vorsicht in den Knochen eingebracht werden müssen. Kommt es beim Einbringen eines entsprechenden Verankerungsteils zu dessen Beschädigung (z.B. ein Teil bricht aus), so ist dessen Entfernung äußerst problematisch. Beispielsweise lässt sich ein derartiges Verankerungsteil nur schwer zerteilen, wobei mit erheblichem Gewebe- und Knochenverlust zu rechnen ist.

Des Weiteren wird das Einbringen des Verankerungsteils meistens durch ein Metallwerkzeug gewährleistet, wobei es bei diesem Vorgang zu einem Abrieb kommen kann, so dass Ablagerungen zurückbleiben. Derartige Ablagerungen können die Herstellung einer Klebeverbindung erheblich stören.

Die EP 1 319 375 A1 schlägt den Einsatz von Epoxidharzen vor, um einen Pin zu realisieren, der in ein Implantat eingesetzt wird und die Verbindung zu einer Krone darstellt. Die verwendeten Epoxidharze können mit entsprechenden Kohlenstoffasern durchzogen oder andere Materialien in diese eingebettet sein.

Die Aufgabe der Erfindung wird durch ein Aufbauteil gemäß dem vorliegenden Anspruch 1 und einem künstlichen Zahnersatz gemäß dem Anspruch 7 gelöst.

Werkzeuge, die zum Einbringen des Verankerungsteils Verwendung finden, sollen also nicht mehr unmittelbar an dem Verankerungsteil ansetzen, sondern an einer hierfür vorgesehenen Werkzeugaufnahme am Aufbauteil, insbesondere Aufbauoberteil. Die aufgebrachten Kräfte (ca. 20 bis 50 Nm, insbesondere 30 bis 40 Nm) werden dann mittelbar an das Verankerungsteil übertragen. Insofern führt beispielsweise ein Abrutschen des Werkzeuges zu einer Beschädigung des Aufbauteils - nicht zu einer Beschädigung des Verankerungsteils. Das Aufbauteil lässt sich wesentlich leichter ersetzen als das Verankerungsteil, so dass eine Schädigung des Patienten vermieden wird. Bei dem Aufbauteil kann es sich um ein Aufbauteil handeln, das später einen Teil des künstlichen Zahnersatzes bildet, oder alternativ um ein Aufbauteil, das nach dem Einbringen des Verankerungsteils entfernt und vorzugsweise durch ein anderes Aufbauteil ersetzt wird. In der vorliegenden Anmeldung kann ein Aufbauteil ein beliebiger 3-dimensionaler Körper sein, der dazu geeignet ist, auf ein Verankerungsteil vorzugsweise formschlüssig aufgesetzt zu werden. Ein entsprechendes Aufbauteil kann lediglich als Einbringhilfe für das Einbringen des Verankerungsteils in das biologische Gewebe verwendet werden. Es ist möglich, jedoch nicht zwingend notwendig, dass das Aufbauteil ein Funktionselement des vollständigen Zahnersatzes, umfassend Krone und Verankerungsteil, bildet.

Die Werkzeugaufnahme kann einen Fortsatz umfassen, der entlang der Längsachse, insbesondere auf der dem Aufbauteil abgewandten Seite auf dem Aufbauoberteil aufsitzt. Die Werkzeugaufnahme steht also über, so dass sie nach einem Einbringen des Verankerungsteils entfernt, z.B. abgeschliffen oder abgeschnitten, werden kann.

Das Aufbauteil weist eine Sollbruchstelle auf, die eine Drehmomentübertragung zwischen Aufbauoberteil und Aufbauunterteil und/oder zwischen Werkzeugaufnahme und Aufbauunterteil beschränkt. Das Aufbauteil weist also eine Kraftübertragungsbeschränkung auf, die das Aufbringen einer zu hohen Kraft auf das Verankerungsteil verhindert. Diese Kraftbeschränkung wird durch eine Sollbruchstelle gewährleistet, die bei der Aufbringung von zu hohen Kräften zu einem kontrollierten Bruch des Aufbauteils führt. Eine Beschädigung des Verankerungsteils kann hierdurch effektiv vermieden werden.

Das Aufbauteil kann mindestens eine Kerbe zur Bereitstellung der Sollbruchstelle umfassen.

Das Profil des Aufbauteils kann ein Mehrkant-Profil und/oder eine Vielrundform umfassen, um das Aufbauteil formschlüssig mit dem Verankerungsteil zu verbinden.

Die Werkzeugaufnahme kann ein Mehrkant-Profil und/oder eine Vielrundform und/oder eine Aufnahme für Mehrkant-Profile und/oder eine Aufnahme für eine Vielrundform umfassen. Theoretisch wäre es denkbar, die Werkzeugaufnahme ähnlich einer Schlitz- oder Kreuzschlitzschraube auszubilden. Zu bevorzugen sind jedoch Vielrundformen oder Mehrkant-Profile. Beispielsweise können Profile verwendet werden, diese von Innentorx- und Außentorx-Schrauben bekannt sind. Alternativ können Mehrkant-Profile (z.B. Innensechskant) verwendet werden. Derartige Profile sind besonders dazu geeignet, hohe Drehmomente zu übertragen, ohne dass es zu einer Beschädigung der entsprechenden Profile - also der Werkzeugaufnahme und somit des Aufbauteils - kommt. Des Weiteren stellen derartige Formen eine bessere Führung des angelegten Werkzeugs bereit. Je nach Anwendung kann eine Propellerform (zweigliedrig und/oder dreigliedrig) gewählt werden. Der Querschnitt kann die Form einer Acht haben.

Das Aufbauteil ist zumindest abschnittsweise beschleifbar ausgebildet sein und umfasst glasfaserverstärkten und/oder kohlenstofffaserverstärkten Kunststoff. Eine derartige Ausbildung ermöglicht es, dass das Aufbauteil an individuelle Gegebenheiten, z.B. durch ein Kürzen, Abtragen des Umfangs, Ausgestalten einer Neigung des Aufbauteils, angepasst werden kann. Theoretisch wäre eine *in situ* Beschleifung denkbar. Kunststoffe sind thermische Isolatoren, so dass auch ein Beschleifen im Mundraum nicht zu einer Erhitzung des Verankerungsteils führt. Die Glasfaser- und/oder Kohlenstofffaserverstärkung führt zu einem sehr stabilen Aufbauteil. Dennoch ist Kunststoff derart flexibel, dass beim Auftreten von übermäßigen Kräften diese nicht unmittelbar an das Verankerungsteil weitergegeben werden.

Die Glasfaser- und/oder Kohlenstofffaserverstärkung istderart, dass die Fasern parallel zu der Längsachse des Aufbauteils ausgerichtet sind. Versuche haben ergeben, dass sich hierdurch ein äußerst stabiles Aufbauteil ergibt, das Rotationskräfte übertragen kann, die größer als 30 Nm sind. Vorzugsweise wird das Aufbauteil im Pultrusionsverfahren hergestellt. Danach kann ein Beschleifen erfolgen.

Das Aufbauunterteil und/oder der Aufbauteilaufnahmebereich kann sich entlang der Längsachse (vorzugsweise nach unten hin) verjüngen.

Das Aufbauoberteil kann gegenüber dem Aufbauunterteil verbreitert und oder nach außen vorspringend zur Ausbildung einer Kontaktfläche ausgebildet sein, wobei sich die Kontaktfläche vorzugsweise im Wesentlichen senkrecht zur Längsachse erstreckt. Der Übergang zwischen Vertikalflächen und Horizontalflächen kann rechtwinklig, spitzwinklig, abgerundet oder stufenförmig ausgestaltet sein. Beispielsweise kann das Aufbauteil insgesamt eine pilzförmige Ausgestaltung haben. Die Kontaktfläche kann dazu verwendet werden, um einen Kontaktschluss zu einer korrespondierenden Fläche am Verankerungsteil herzustellen. Insofern ist es möglich, eine Klebeverbindung zwischen dem Aufbauteil und dem Verankerungsteil herzustellen.

Das Aufbauteil/die Einbringhilfe kann einen soliden Grundkörper aus Kunststoff, insbesondere glasfaserverstärktem und/oder kohlenstofffaserverstärkem Kunststoff, umfassen. Ein solider Körper ist besonders dazu geeignet, die aufgebrachten Kräfte (bei einer technischen Keramik 20-35 Nm, bei Metall 20-50 Nm) zu übertragen. Bei dem glasfaserverstärkten Kunststoff kann es sich um einen Faser-Kunststoff-Verbund handeln, wobei als Basis Epoxid-Harz verwendet wird. In einem Ausführungsbeispiel kann der Faseranteil in dem glasfaserverstärkten und/oder kohlenstofffaserverstärkten Kunststoff größer als 50% und/oder größer als 60% und/oder größer als 70% sein. Es hat sich herausgestellt, dass ein besonders hoher Faseranteil dazu führt, dass bei einem Überdrehen - also bei einem Aufbringen einer zu hohen Kraft - eine visuell gut wahrnehmbare Verfärbung des Materials auftritt. Dies kann vom Arzt als Signal verstanden werden, dass das Aufbauteil unbrauchbar ist. Des Weiteren führt der hohe Faservolumenanteil dazu, dass sogar bei einer soliden Ausbildung des Aufbauteils eine relativ definierte Widerstandsfähigkeit etabliert werden kann. Insofern kann das Aufbauteil derart dimensioniert werden, dass bei einer vordefinierten Kraft (z.B. bei 35, 40 oder 45 Nm) eine Materialermüdung auftritt. Insofern wird die Kraftübertragung jenseits dieser Grenzen effektiv verhindert.

Des Weiteren kann das Aufbauteil derart ausgebildet sein, dass es formschlüssig in das hierfür ausgebildete Verankerungsteil einsteckbar ist.

Die oben genannte Aufgabe wird des Weiteren durch einen künstlichen Zahnersatz mit einem Implantat zur Aufnahme einer Krone gelöst, wobei das Implantat ein Aufbauteil wie das vorhergehend beschriebene und ein Verankerungsteil aufweist. Das Verankerungsteil kann einen Aufbauteilaufnahmebereich zur Aufnahme des Aufbauteils aufweisen und zumindest abschnittsweise aus einem ersten Material ausgebildet sein, wobei das erste Material vorzugsweise zur Werkstoffgruppe der technischen Keramiken, insbesondere der Oxidkeramik, gehört. Es ergeben sich ähnliche Vorteile, wie diese bereits in Verbindung mit dem Aufbauteil beschrieben wurden.

Das Verankerungsteil kann einen Schulter-Abschnitt bzw. frustokonischen Abschnitt, insbesondere mit einer konkaven Mantelfläche, zur Aufnahme eines Teils einer Krone aufweisen. Durch die besondere Ausgestaltung kann ein Kronenaufnahmebereich hergestellt werden. Dieser ist besonders dafür geeignet, eine Präparationsgrenze zu bilden, so dass die Krone auf das Verankerungsteil und optional das Aufbauteil aufgetragen werden kann. Der Kronenaufnahmebereich stellt sicher, dass bei diesem materialauftragenden Vorgang keine Hohlräume oder Überstände entstehen, die einen bakteriellen Befall unterstützen.

Das Verankerungsteil kann mindestens einen Gewindeabschnitt zum Eindrehen des Verankerungsteils in einen Knochen umfassen. Vorzugsweise lässt sich also das Verankerungsteil über ein Gewinde in den Knochen verankern. Das Einbringen des Verankerungsteils wird dadurch erleichtert, dass Gewindeabschnitte vorgesehen sind, die das Eindrehen des Verankerungsteils nach Art einer Schraube ermöglichen. Die erfindungsgemäße Ausgestaltung des Aufbauteils mit der Werkzeugaufnahme kommt an dieser Stelle besonders zum Tragen, da das Drehmoment dazu genutzt werden kann, das Verankerungsteil in den Knochen einzudrehen.

Das Verankerungsteil kann derart ausgebildet sein, dass zumindest eine Querschnittsfläche eine im Wesentlichen ovale, insbesondere elliptische, Flächenbegrenzung aufweist. Diese Querschnittsfläche ergibt sich vorzugsweise bei einem Schnitt durch das Verankerungsteil senkrecht zu dessen Längsachse. Je nachdem, welchen Zahn der künstliche Zahnersatz ersetzen soll, ist es wünschenswert, Verankerungsteile in unterschiedlicher Ausgestaltung bereit zu stellen. Beispielsweise bleibt bei einem Ersatz eines Prämolars nur wenig Platz zwischen den Nachbarzähnen für das Verankerungsteil. Daher muss das Verankerungsteil sehr klein, beispielsweise mit einem Durchmesser des Subgingival-Abschnitts kleiner als 5 mm, insbesondere kleiner als 4,5 mm, ausgestaltet werden. Um die Krone in geeigneter Weise aufmodellieren und/oder aufsetzen zu können, wird im oberen Bereich (z.B. isogingival und/oder im Schulter-Abschnitt) eine Verbreiterung vorgenommen. Um hier den natürlichen Gegebenheiten Rechnung zu tragen, kann diese Verbreiterung in einer Draufsicht eine im Wesentlichen ovale Ausgestaltung haben. Vorzugsweise ist das Verankerungsteil derart ausgebildet, dass sich insbesondere im oberen Bereich eine Querschnittsfläche ergibt, die achsensymmetrisch zu zumindest einer Symmetrieachse ist, die sich im eingesetzten Zustand von der pallatinalen Seite des künstlichen Zahnersatzes zu dessen labialen Seite erstreckt.

Insbesondere bei derartig kleinen Verankerungsteilen ist es vorteilhaft, wenn der Aufbauteilaufnahmebereich einen länglichen Schlitz, insbesondere ein Langloch umfasst. Dieses Langloch kann sich entlang der besagten Symmetrieachse erstrecken. Bei einem sehr kleinen Verankerungsteil ist es somit möglich, relativ hohe Torsionskräfte beim Einbringen zu übertragen. Des Weiteren hat diese Ausgestaltung den Vorteil, dass Kräfte, die auf die Krone wirken, in geeigneter Weise in das Verankerungsteil eingeleitet werden können. Insbesondere ergibt sich eine hohe Steifigkeit entlang der besagten Symmetrieachse, so dass die üblichen Kräfte optimal abgeleitet werden können.

Wenn das Verankerungsteil einen Gewindeabschnitt umfasst, wie dieser bereits beschrieben wurde, sollte das Gewinde mit einer relativ geringen Gewindesteigung ausgestattet sein. Vorzugsweise ist das Gewinde derart ausgestaltet, dass sich pro Umdrehung ein Höhenunterschied von weniger als 2 mm, insbesondere weniger als 1 mm, ergibt. Insofern ist es möglich, das Verankerungsteil vorteilhaft (z.B. wegen einer vorgegebenen Ausrichtung des Verankerungsteils und/oder der Aufbauteilaufnahmebereich) auszurichten. Insofern kann ein optimaler Sitz des Verankerungsteils gewährleistet werden, wobei beispielsweise eine 180°-Drehung beim Einsetzen nur zu einem geringen Höhenunterschied führt.

Weitere vorteilhafte Ausführungsformen ergeben sich anhand der Unteransprüche.

Nachfolgend wird die Erfindung mittels mehrerer Ausführungsbeispiele beschrieben, die anhand von Abbildungen näher erläutert werden. Hierbei zeigen:
- Fig. 1: einen Schnitt durch einen erfindungsgemäßen künstlichen Zahnersatz mit einer Krone, einem Verankerungsteil und einem Aufbauteil;
- Fig. 2: eine Draufsicht auf das Verankerungsteil aus Fig. 1;
- Fig. 3: eine erste Ausführungsform eines Aufbauteils;
- Fig. 4: eine zweite Ausführungsform eines Aufbauteils mit einer Werkzeugaufnahme in Form eines Kleeblatts;
- Fig. 5: eine dritte Ausführungsform eines Aufbauteils mit einem Dreikant als Werkzeugaufnahme;
- Fig. 6: eine vierte Ausführungsform eines Aufbauteils mit einem Dreikant als Werkzeugaufnahme;
- Fig. 7: eine fünfte Ausführungsform eines Aufbauteils (quadratisch);
- Fig. 8: eine sechste Ausführungsform eines Aufbauteils (konisch);
- Fig. 9: das eingesetzte Verankerungsteil aus Fig. 1;
- Fig. 10: eine Draufsicht auf ein weiteres Verankerungsteil ;
- Fig. 11: eine siebte Ausführungsform eines Aufbauteils (elliptisch); und
- Fig. 12: das eingesetzte Verankerungsteil aus Fig. 10.

In der nachfolgenden Beschreibung werden für gleiche und gleich wirkende Teile dieselben Bezugsziffern verwendet.

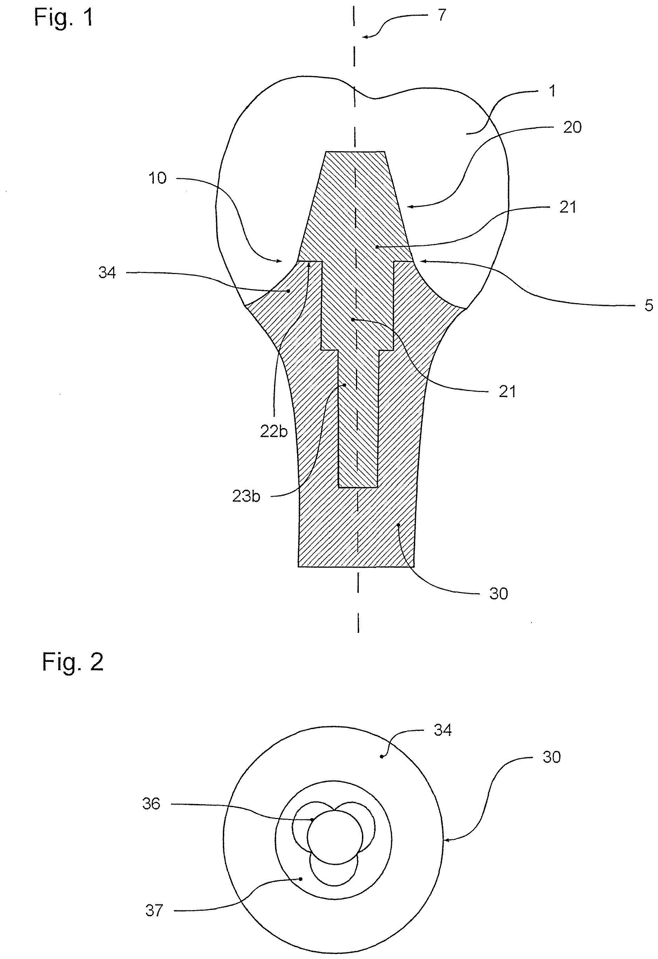

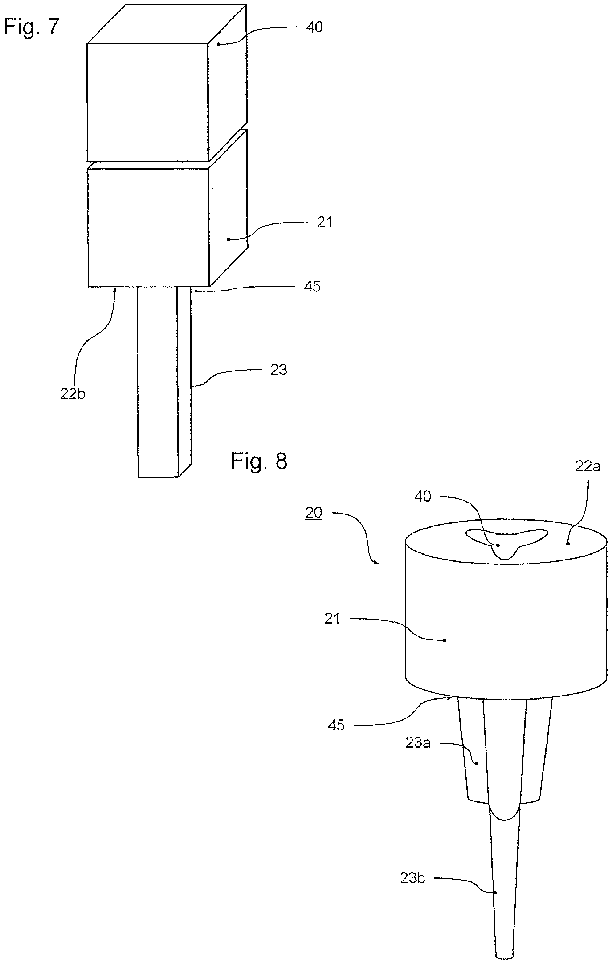

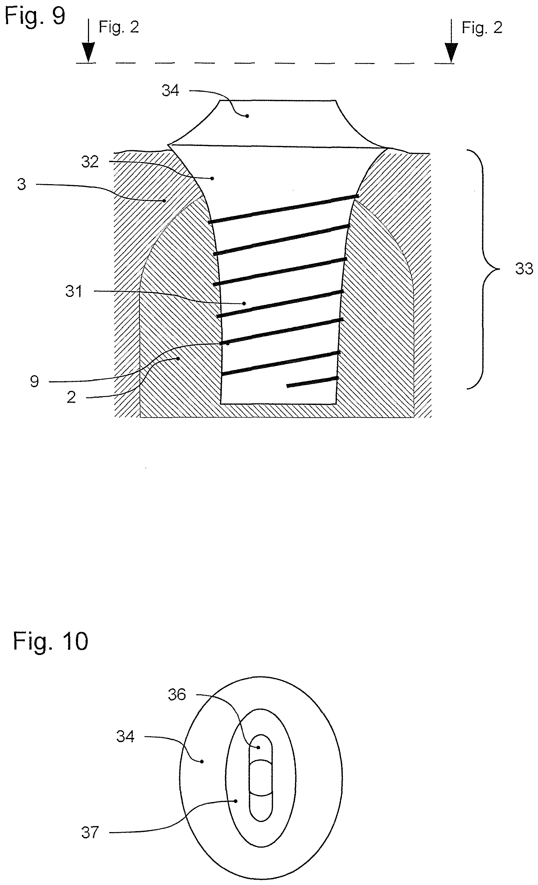

Ein erfindungsgemäßer künstlicher Zahnersatz umfasst, wie aus Fig. 1 ersichtlich, eine Krone 1, ein Aufbauteil 20 und ein Verankerungsteil 30, wobei das Aufbauteil 20 und das Verankerungsteil 30 ein zweiteiliges Implantat 10 ausbilden, auf dem die Krone 1 aufsitzt. Wie aus Fig. 2 und 8 ersichtlich, ist das Verankerungsteil 30 im beschriebenen Ausführungsbeispiel ein im Wesentlichen rotationssymmetrischer Stift, der sich entlang einer Längsachse 7 erstreckt und in das Zahnfleisch 3 und den Knochen 2 eingesetzt wird. In einem weiteren Ausführungsbeispiel kann das Verankerungsteil 30 asymmetrisch ausgeführt und an individuelle Gegebenheiten angepasst sein. Gemäß der Seitenansicht (vgl. die schematische Darstellung aus Fig. 9) nimmt der Durchmesser des Verankerungsteils 30 in einem Subgingival-Abschnitt 33 von unten her zu und jenseits des Subgingival-Abschnitts 33 ab.

Dieser Abschnitt jenseits des Subgingival-Abschnitts 33 wird als Schulter-Abschnitt 34 bezeichnet, der in einem Plateau-Abschnitt endet, der als Aufnahmebereich 37 bezeichnet ist. Vorzugsweise ist das Verankerungsteil 30 patientenspezifisch derart individualisiert, dass der Übergang zwischen Subgingival-Abschnitt 33 und Schulter-Abschnitt 34 isogingival verläuft.

Der frustokonische Schulter-Abschnitt 34 hat eine konkave Mantelfläche, die die Krone 1 aufnimmt.

Wie aus Fig. 1 ersichtlich, liegt auf dem Plateau-Abschnitt bzw. Aufnahmebereich 37 ein Teilabschnitt des Aufbauteils 20 auf. Der Aufnahmebereich 37 kann beim Setzten des Implantats mit dem Aufbauteil 20 verklebt werden.

Entlang der Längsachse 7 erstreckt sich im Inneren des Verankerungsteils 30 ein Aufnahmekanal 36, der ebenfalls einen Abschnitt des Aufbauteils 20 aufnimmt.

In einem ersten Ausführungsbeispiel ist der obere Abschnitt des Aufnahmekanals 36 als Vielrundform und der untere Abschnitt als Zylinder ausgebildet. Im Endeffekt hat der obere Abschnitt des Aufnahmekanals 36 im Querschnitt eine Ausgestaltung, die drei sich überschneidenden Kreisen - ähnlich einem Kleeblatt - entspricht. Der entsprechende Querschnitt durch den unteren Abschnitt des Aufnahmekanals 36 ist kreisförmig.

Das Aufbauteil 20 umfasst ein korrespondierend zu dem Aufnahmekanal 36 ausgebildetes Aufbauunterteil 23, auf dem ein Aufbauoberteil 21 aufsitzt. Das Aufbauunterteil 23 ist in ein Antriebsteil 23a (korrespondierend zum oberen Abschnitt des Aufnahmekanals 36) und in ein Retentionsteil 23b (korrespondierend zum unteren Abschnitt des Aufnahmekanals 36) unterteilt. Im Querschnitt ragt der Antriebsteil 23a des Aufbauunterteils 23 gegenüber dem Retentionsteil 23b heraus, wobei dieser von dem Aufbauoberteil 21 überragt wird. Im vollständig implantierten Zustand fluchtet das frustokonisch ausgebildete Aufbauoberteil 21 mit dem Schulter-Abschnitt 34, insbesondere der konkaven Mantelfläche, des Verankerungsteils 30 und liegt mittels einer Aufbauoberteil-Bodenfläche 22b auf dem Aufnahmebereich 37 des Verankerungsteils 30 auf. Das einstückig ausgebildete Aufbauteil 20 bildet mit dem Verankerungsteil 30 eine Kontaktfuge 5, die durch die Krone 1 überdeckt und verschlossen ist.

In einem bevorzugten Ausführungsbeispiel ist das Verankerungsteil 30 als technische Keramik und das Aufbauteil 20 aus Kunststoff ausgebildet. Auch für die Herstellung der Krone 1 kann eine technische Keramik, beispielsweise Zirkonoxid, verwendet werden. Insofern hat der künstliche Zahnersatz ein flexibles "Rückgrat" in Form des Aufbauteils 20 und eine starre Schale in Form von der Krone 1 und dem Verankerungsteil 30.

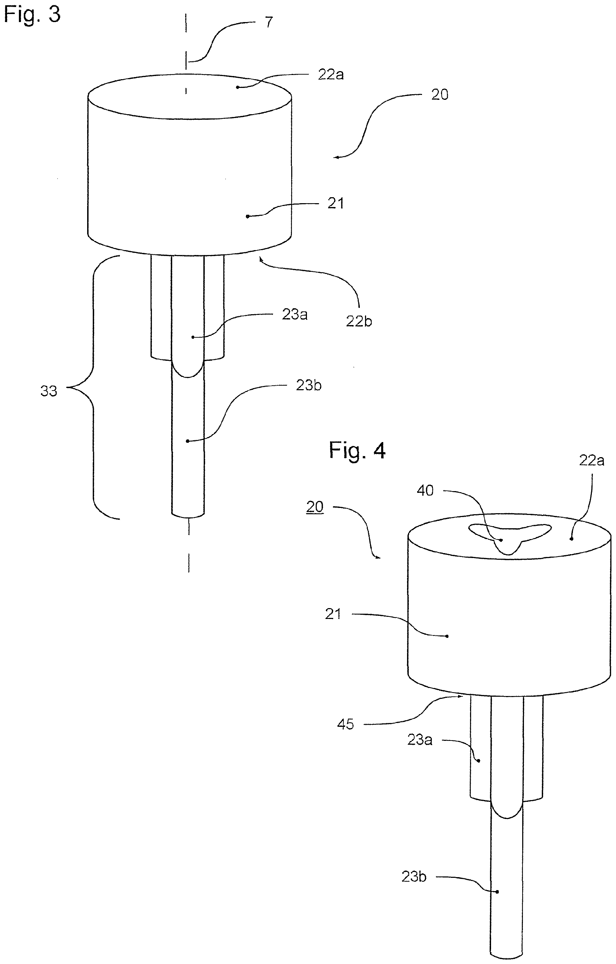

Das Aufbauteil lässt sich vorzugsweise an patientenspezifische Gegebenheiten anpassen. Anhand der Fig. 3 wird erläutert, wie sich ein entsprechend individualisiertes Aufbauteil 20 herstellen lässt. In einer Ausgangsform hat das Aufbauteil 20, wie bereits erläutert, ein Aufbauoberteil 21 und ein Aufbauunterteil 23 mit einem Antriebsteil 23a und einem Retentionsteil 23b. Das Aufbauoberteil 21 ist als Zylinder ausgeführt und derart bemessen, dass es den Aufnahmebereich 37 seitlich überragt. Der Zylinder hat eine Aufbauoberteil-Deckelfläche 22a und die Aufbauoberteil-Bodenfläche 22b. Nach der Implantation des Verankerungsteils 30 wird das Aufbauteil 20 mit dem korrespondierend zu dem Aufnahmekanal 36 ausgebildeten Antriebsteil 23a und Retentionsteil 23b in den Aufnahmekanal 36 eingesteckt. Die Aufbauoberteil-Bodenfläche 22b und der Aufnahmebereich 37 kontaktieren sich im eingesteckten Zustand des Aufbauteils 20. Nach dem Einstecken kann das Aufbauoberteil 21 *in situ* oder in einem Modell derart angepasst werden, dass patientenspezifische Gegebenheiten berücksichtigt werden. Beispielsweise kann das Aufbauteil 20, insbesondere das Aufbauoberteil 21, derart beschliffen werden, dass sich eine frustokonische Form ergibt, wie diese in Fig. 1 gezeigt wird.

In einer alternativen Ausführungsform können vorkonfektionierte Aufbauteile 20 bereitgestellt werden.

Ein wesentlicher Teil der vorliegenden Anmeldung beschäftigt sich mit dem effektiven Einsetzen des Verankerungsteils 30 in den Knochen 2 und das Zahnfleisch 3. Hierfür weist das Verankerungsteil 30, wie in der Fig. 9 gezeigt, einen Gewindeabschnitt 31 auf, der es ermöglicht, das Verankerungsteil 30 in den Knochen 2 einzudrehen. Es ist möglich, Werkzeuge bereitzustellen, die in den Aufnahmekanal 36 des Verankerungsteils 30 eingreifen und das Eindrehen erleichtern. Hierbei kann es jedoch leicht zu einer Beschädigung des Verankerungsteils 30 kommen. Des Weiteren können so hohe Kräfte aufgebracht werden, dass der Knochen 2 des Patienten geschädigt wird.

Die vorliegende Erfindung löst dieses Problem dadurch, dass vor dem Eindrehen des Verankerungsteils 30 in den Knochen 2 ein Aufbauteil wie beispielsweise in Fig. 4 gezeigt, eingesetzt wird. Aufgrund des Formschlusses des Retentionsteils 23b mit dem Aufnahmekanal 36 entsteht so eine kraftschlüssige Verbindung zwischen dem Aufbauteil 20 und dem Verankerungsteil 30. Der Kraftschluss wird insbesondere bezüglich einer Rotationsbewegung um die Längsachse 7 hergestellt. Erfindungsgemäß wird eine Werkzeugaufnahme 40 am Aufbauteil 20 vorgesehen, die ein insbesondere formschlüssiges Ansetzen des Werkzeugs ermöglicht. Im in Fig. 4 gezeigten Ausführungsbeispiel handelt es sich bei der Werkzeugaufnahme 40 um eine an der Aufbauoberteil-Deckelfläche 22a vorgesehene Aussparung, die im Endeffekt eine ähnliche Ausgestaltung hat, wie der obere Bereich des Aufnahmekanals 36. Die Aussparung ist also in Form einer Vielrundform ausgebildet, die ähnlich aussieht, wie drei sich überschneidende Bohrungen bzw. ein Kleeblatt. Der hier tätige Arzt kann also das gleiche Instrument, das ursprünglich zur unmittelbaren Eindrehung des Verankerungsteils 30 verwendet wurde, verwenden, um das Verankerungsteil 30 mittels des erfindungsgemäßen Aufbauteils 20 einzudrehen.

In einem Ausführungsbeispiel weist das erfindungsgemäße Aufbauteil 20 eine Sollbruchstelle 45 auf, die sich zwischen dem Aufbauoberteil 21 und dem Aufbauunterteil 23, insbesondere oberhalb des Antriebsteils 23a befindet. Diese Sollbruchstelle 45 kann derart ausgebildet sein, dass beim Auftreten von für das Verankerungsteil 30 schädlichen Kräften das Aufbauoberteil 21 abbricht. Eine Beschädigung des Verankerungsteils 30 wird also effektiv verhindert. Der nach einem entsprechenden Bruch im Verankerungsteil 30 verbleibende Aufbauunterteil 23, der vorzugsweise aus Kunststoff ist, lässt sich einfach entfernen.

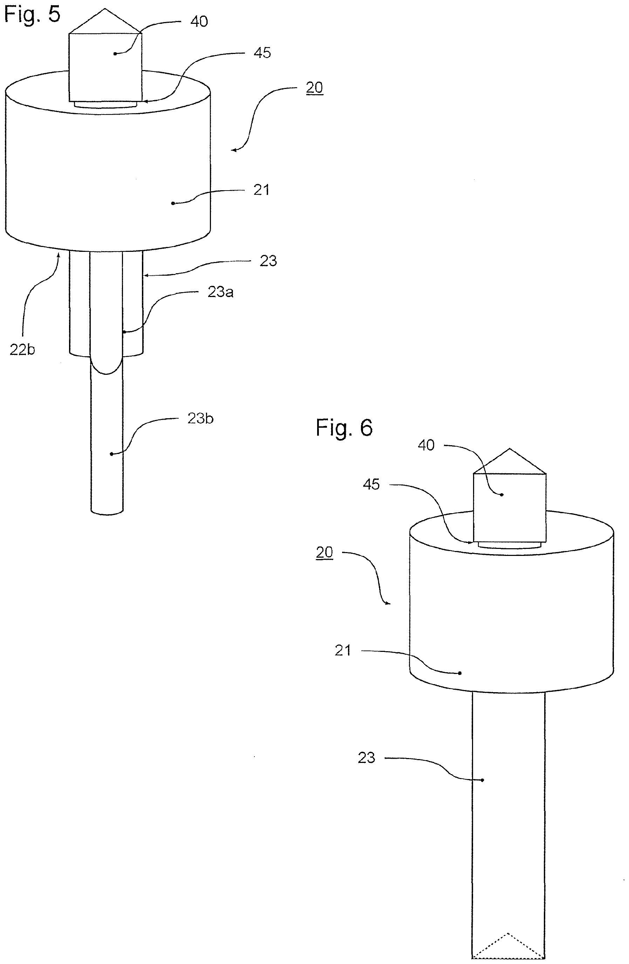

In einem weiteren Ausführungsbeispiel (vgl. Fig. 5) ist die Werkzeugaufnahme 40 nicht in das Aufbauoberteil 21 eingelassen, sondern ragt über dieses heraus. Die Fig. 5 zeigt einen Dreikant, der über die Aufbauoberteil-Deckelfläche 22a übersteht und von einem korrespondierend ausgebildeten Werkzeug (z.B. einem Dreikantschlüssel) aufgenommen werden kann.

Eine Kerbe zwischen der Werkzeugaufnahme 40 aus Fig. 5 und dem Aufbauoberteil 21 bildet die Sollbruchstelle 45, die kontrolliert abbrechen kann. Die Ausführungsform gemäß Fig. 5 hat den Vorteil, dass auch nach dem Abbrechen der Werkzeugaufnahme 40 ein voll funktionsfähiges Aufbauteil 20 verbleibt. Insofern kann ein Abbrechen der Werkzeugaufnahme 40 bewusst herbeigeführt werden. Alternativ kann nach dem erfolgreichen Einbringen des Verankerungsteils 30 der Überstand in Form der Werkzeugaufnahme 40 abgeschliffen werden. Gegebenenfalls kann eine Nachbearbeitung des Aufbauoberteils 21 erfolgen. Insofern ist es möglich, das Aufbauteil 20 nicht nur als Einsetzhilfe, sondern auch als Bestandteil des künstlichen Zahnersatzes zu verwenden.

Während in Fig. 5 das Antriebsteil 23a und das Retentionsteil 23b den Antriebsteilen 23a und Retentionsteilen 23b der Ausführungsbeispiele gemäß den Fig. 3 und 4 gleichen, ist in Fig. 6 ein Aufbauunterteil 23 vorgesehen, das eine dreieckige Grundfläche aufweist. Das Aufbauunterteil 23 gemäß Fig. 6 ist also ein langgezogener Dreikant, der ähnlich wie die Vielrundform aus den Fig. 3 bis 5 in einen korrespondierenden Aufnahmekanal 36 eingebracht werden kann. Auch hier ergibt sich ein Formschluss, der ein effektives Übertragen der auf das Aufbauteil 20 ausgeübten Kräfte auf das Verankerungsteil 30 gewährleistet.

Fig. 7 zeigt ein weiteres Ausführungsbeispiel des erfindungsgemäßen Aufbauteils 20. Das Aufbauoberteil 21 und das Aufbauunterteil 23 sind in Form eines Quaders ausgebildet. Die Grundfläche der Quader sind Quadrate, wobei der Quader des Aufbauunterteils 23 mittig an der Aufbauoberteil-Bodenfläche 22b ansetzt. Das Aufbauunterteil 23 bildet also einen Vierkant, der in einen korrespondierend ausgebildeten Aufnahmekanal 36 formschlüssig eingebracht werden kann. Das quaderförmige Aufbauoberteil 21 kann, wie die bereits erläuterten Aufbauoberteile 21 beschliffen werden. In der unbeschliffenen Form bildet das gesamte Aufbauoberteil 21 jedoch ebenfalls einen Vierkant aus, der als Werkzeugaufnahme 40 für einen korrespondierend ausgebildeten Werkzeugschlüssel dienen kann. Im Endeffekt greift der Schlüssel an mindestens zwei wechselseitig angeordneten Seitenflächen des Quaders des Aufbauoberteils 21 formschlüssig an, so dass sich ein Drehmoment aufbringen lässt. Das Aufbauoberteil 21 bildet also die Werkzeugaufnahme 40, die zum Aufbringen von geeigneten Kräften verwendet wird. Es lässt sich also auch mittels des Aufbauteils 20 gemäß der Fig. 7 ein entsprechend ausgebildetes Verankerungsteil 30 in einen Knochen 2 einschrauben. Vorzugsweise setzt sich das Aufbauteil 21 aus zwei Würfeln zusammen, wobei diese unter Bildung einer Sollbruchstelle 45 miteinander verbunden sind. Soweit der Werkzeugschlüssel also im oberen Bereich des Aufbauteils 21 angesetzt wird, lässt sich die auf das Verankerungsteil 30 übertragene Kraft effektiv begrenzen.

Fig. 8 zeigt ein weiteres Ausführungsbeispiel des erfindungsgemäßen Aufbauteils 20. Gegenüber dem Aufbauteil aus Fig. 4 ist hier das Aufbauunterteil 23 insgesamt konisch ausgebildet, so dass der Durchmesser des Aufbauunterteils 23 von oben nach unten hin abnimmt. Sowohl das Antriebsteil 23a mit der kleeblattförmigen Ausgestaltung als auch das Retentionsteil 23b kann sich nach unten hin verjüngen.

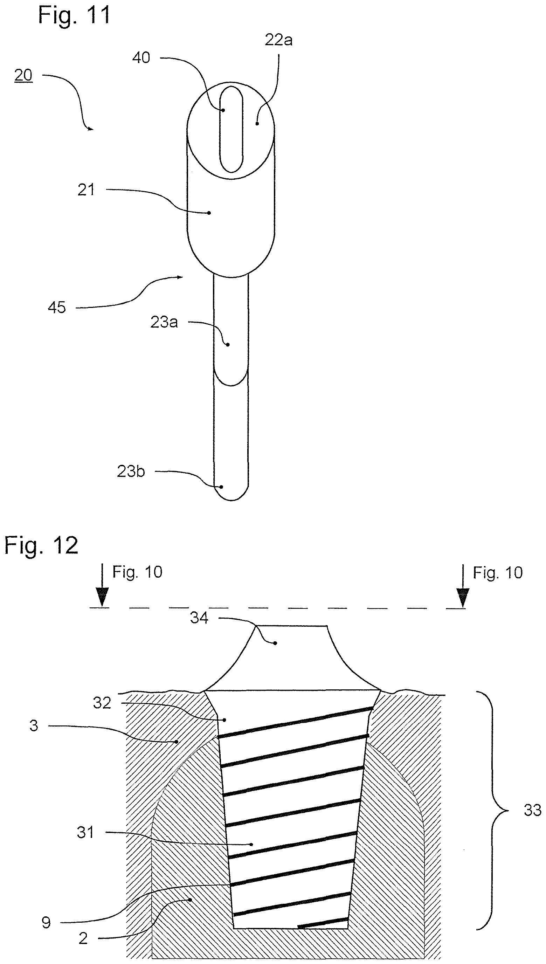

Fig. 11 zeigt ein weiteres erfindungsgemäßes Aufbauteil 20, das bezüglich dessen Längsachse 7 keine rotationssymmetrische Ausbildung hat. Im Endeffekt ist das Aufbauoberteil 21 ein endlicher Zylinder mit einer elliptischen Aufbauoberteil-Deckelfläche 22a und einer elliptischen Aufbauoberteil-Bodenfläche 22b. Als Werkzeugaufnahme 40 ist ein Langloch vorgesehen, dass derart in dem Zylinder angeordnet ist, dass die Länge des Langlochs maximiert werden kann, wobei in den Randbereichen ausreichend Material verbleibt, so dass beim Einsetzen eines Werkzeugs keine Beschädigung des Aufbauteils 20 auftritt. Auch das Aufbauunterteil 23 weist ein korrespondierend ausgebildetes Antriebsteil 23a auf, das ebenfalls als Zylinder mit elliptischen, vorzugsweise ovalen Boden- und Deckelflächen ausgebildet ist. Das Retentionsteil 23b kann einen kreisförmigen Querschnitt oder auch jeden beliebigen anderen Querschnitt, insbesondere einen elliptischen haben.

Ein entsprechendes Aufbauteil 20 kann in besonders vorteilhafter Weise in Verbindung mit einem langgezogenen Verankerungsteil 30 verwendet werden, wie dies in den Fig. 10 und 12 gezeigt ist. Ein entsprechendes Verankerungsteil 30 hat einen zylinderförmigen unteren Subgingival-Abschnitt 33 . Im oberen Subgingival-Abschnitt 33 weitet sich das Verankerungsteil 30 auf, so dass sich hier ein Kegelstumpf mit einer elliptischen Deckelfläche ergibt. Dementsprechend ist auch der Schulter-Abschnitt 34 und der Aufnahmebereich 37 entsprechend elliptisch ausgebildet. Der Aufnahmekanal 36 kann dann in Form eines Langlochs ausgestaltet sein, das besonders dazu geeignet ist, Torsionskräfte aufzunehmen. Insofern lässt sich in einfacher Weise eine Kräfteübertragung zwischen dem Aufbauteil 20 aus Fig. 11 und dem Verankerungsteil 30 aus den Fig. 10 und 12 herstellen.

Allgemein besteht im Bereich der Zahnmedizin das Problem, dass eindrehbare Verankerungsteile 30 sehr klein dimensioniert sein können. Beispielsweise kann der untere Subgingival-Abschnitt 33 einen Durchmesser von weniger als 5 mm, insbesondere weniger als 4 mm, insbesondere weniger als 3 mm, aufweisen. Es erweist sich als äußerst problematisch, an derart kleinen Verankerungsteilen 30 einen Aufnahmekanal 36 vorzusehen, der dazu geeignet ist, ausreichend hohe Kräfte, z.B. größer als 30 Nm aufzunehmen. Die vorliegende Erfindung schlägt daher vor, das Verankerungsteil 30 mit einem langgezogenen oberen Bereich (z.B. einem langgezogenen Schulter-Abschnitt 34) zu versehen. Derartig ausgestaltete Verankerungsteile 30 können besonders geeignet sein, um künstliche Zahnersätze für Prämolare herzustellen. Des Weiteren eigenen sie sich für die Anbringung von schlitzförmigen Öffnungen, wie diese z.B. in Fig. 10 gezeigt sind. Die erfindungsgemäße schlitzförmige Öffnung, vorzugsweise in Form eines Langlochs, vereinfacht das Eindrehen der Verankerungsteile 30 in den Knochen 2.

Anhand der beschriebenen Ausführungsbeispiele sollte es klar sein, dass das Aufbauunterteil 23 oder ein Teilbereich dessen (z.B. das Antriebsteil 23a) sehr unterschiedliche Formen aufweisen kann, die das erfindungsgemäße Ziel, nämlich einen Formschluss mit einem korrespondierend ausgebildeten Aufnahmekanal 36 bewerkstelligen. Denkbar sind Mehrkant-Profile, z.B. Dreikant, Vierkant, Fünfkant, Sechskant, usw. oder Vielrundformen, z.B. wie diese von Torx-Schraubenbits bekannt sind.

Des Weiteren gibt es zahlreiche unterschiedliche Möglichkeiten, wo die Werkzeugaufnahme 40 am Aufbauteil 20 vorgesehen wird. Wie beschrieben, kann die Werkzeugaufnahme 40 in das Aufbauoberteil 21 eingelassen (z.B. Fig. 4) und/oder an diesem befestigt (z.B. Fig. 5) sein. Des Weiteren kann das Aufbauoberteil 21 eine Form aufweisen, die die Funktionalität einer Werkzeugaufnahme 40 bereitstellt.

Des Weiteren kann die Sollbruchstelle 45 je nach Anforderung an unterschiedlichen Positionen vorgesehen werden. So ist es beispielsweise denkbar, die Sollbruchstelle 45 bei dem Aufbauteil 20 aus Fig. 4 nicht zwischen dem Aufbauoberteil 21 und dem Aufbauunterteil 23 vorzusehen, sondern stattdessen eine entsprechende Sollbruchstelle mittig, wie beispielsweise in Fig. 7 gezeigt, am Aufbauoberteil 21 vorzusehen. Insofern ist es möglich, ein Aufbauteil 20 zu schaffen, das auch nach dem Auslösen des Sollbruchs funktionell zur Schaffung eines künstlichen Zahnersatzes eingesetzt werden kann. Bezüglich der Ausgestaltung des Aufbauoberteils 21 ergeben sich zahlreiche Variationsmöglichkeiten. Beispielsweise kann das Aufbauoberteil 21 aus Fig. 4 derart dimensioniert werden, dass nach dem Sollbruch oder nach der Abnahme der Werkzeugaufnahme 40 ein Aufbauoberteil 21 verbleibt, wie dieses in Fig. 3 gezeigt ist. Des Weiteren kann die Sollbruchstelle 45 je nach Anforderung an unterschiedlichen Positionen z.B. am Aufbauoberteil 21 vorgesehen sein. Beispielsweise kann sie sich mittig, wie in Fig. 7 gezeigt, oder im unteren oder im oberen Drittel des Aufbauoberteils 21 befinden. Es sollte für den hier tätigen Fachmann klar sein, dass die Position der Sollbruchstelle 45 maßgeblich dafür verantwortlich ist, wie das Aufbauteil 20 nach einem Sollbruch aussieht.

In den vorhergehenden Ausführungsbeispielen wurde das erfindungsgemäße Aufbauteil 20 in Verbindung mit einer Krone 1 beschrieben. An Stelle der Krone 1 können beliebige Suprakonstruktionen durch das beschriebene Aufbauteil gestützt werden.

### Bezugszeichenliste

- 1: Krone
- 2: Knochen
- 3: Zahnfleisch
- 5: Kontaktfuge
- 7: Längsachse
- 9: Gewinde
- 10: Implantat
- 20: Aufbauteil
- 21: Aufbauoberteil
- 22a: Aufbauoberteil-Deckelfläche
- 22b: Aufbauoberteil-Bodenfläche
- 23: Aufbauunterteil
- 23a: Antriebsteil
- 23b: Retentionsteil
- 30: Verankerungsteil
- 31: Gewindeabschnitt
- 32: gewindefreier Abschnitt
- 33: Subgingival-Abschnitt
- 34: Schulter-Abschnitt
- 36: Aufnahmekanal
- 37: Aufnahmebereich
- 40: Werkzeugaufnahme
- 45: Sollbruchstelle

## Patentansprüche

1. Aufbauteil (20) für einen künstlichen Zahnersatz, umfassend:
- ein entlang einer Längsachse (7) angeordnetes Aufbauoberteil (21) und
- ein Aufbauunterteil (23), das zum formschlüssigen Einsetzen des Aufbauteils (20) in einen Aufbauteilaufnahmebereich (26) eines Verankerungsteils (30) ein derartiges Profil aufweist, dass ein auf das Aufbauteil (20) aufgebrachtes Drehmoment auf das Verankerungsteil (30) übertragbar ist,
wobei das Aufbauoberteil (21) eine Werkzeugaufnahme (40) zur formschlüssigen Aufnahme eines Werkzeugs umfasst wobei das Aufbauteil (20) zumindest abschnittsweise beschleifbar, als glasfaserverstärkter und/oder kohlenstofffaserverstärkter Kunststoff ausgebildet ist, wobei die Fasern parallel zur Längsachse (7) ausgerichtet sind
**dadurch gekennzeichnet, dass**
eine Sollbruchstelle (45) die Drehmomentübertragung zwischen Aufbauoberteil (21) und Aufbauunterteil (23) und/oder zwischen Werkzeugaufnahme (40) und Aufbauunterteil (23) beschränkt.

2. Aufbauteil (20) nach Anspruch 1,
**dadurch gekennzeichnet, dass**
die Werkzeugaufnahme (40) einen Fortsatz umfasst, der entlang der Längsachse (7), insbesondere auf der dem Aufbauunterteil (23) abgewandten Seite, auf dem Aufbauoberteil (21) aufsitzt.

3. Aufbauteil (20) nach einem der vorhergehenden Ansprüche,
**gekennzeichnet durch**
mindestens eine Kerbe zur Bereitstellung einer/der Sollbruchstelle (45).

4. Aufbauteil (20) nach einem der vorhergehenden Ansprüche,
**dadurch gekennzeichnet, dass**
das Profil des Aufbauteils (20) ein Mehrkant-Profil und/oder eine Vielrundform umfasst.

5. Aufbauteil (20) nach einem der vorhergehenden Ansprüche,
**dadurch gekennzeichnet, dass**
die Werkzeugaufnahme (40) ein Mehrkant-Profil und/oder eine Vielrundform und/oder eine Aufnahme für ein Mehrkant-Profil und/oder eine Aufnahme für eine Vielrundform umfasst.

6. Aufbauteil (20) nach einem der vorhergehenden Ansprüche,
**dadurch gekennzeichnet, dass**
das Aufbauoberteil (21) gegenüber dem Aufbauunterteil (23) verbreitert und/oder nach außen vorspringend zur Ausbildung einer Kontaktfläche (22b) ausgebildet ist, wobei sich die Kontaktfläche vorzugsweise im Wesentlichen senkrecht zur Längsachse (7) erstreckt.

7. Künstlicher Zahnersatz mit einem Implantat (10) zur Aufnahme einer Krone (1), wobei das Implantat (10) ein Aufbauteil (20) nach einem der vorhergehenden Ansprüche und ein Verankerungsteil (30) aufweist,
wobei das Verankerungsteil (30) einen Aufbauteilaufnahmebereich (36) zur Aufnahme des Aufbauteils (20) aufweist und zumindest abschnittsweise aus einem ersten Material ausgebildet ist, wobei das erste Material vorzugsweise zur Werkstoffgruppe der technischen Keramik gehört.

8. Künstlicher Zahnersatz nach Anspruch 7,
**dadurch gekennzeichnet, dass**
das Verankerungsteil (30) einen frustokonischen Abschnitt (34) mit einer konkaven Mantelfläche zur Aufnahme eines Teils einer Krone (1) aufweist.

9. Künstlicher Zahnersatz nach Anspruch 7 oder 8,
**dadurch gekennzeichnet, dass**
das Verankerungsteil (30) mindestens einen Gewindeabschnitt (31a, 31b) zum Eindrehen des Verankerungsteils (30) in einen Knochen (2) umfasst.

10. Künstlicher Zahnersatz nach Anspruch 9,
**dadurch gekennzeichnet, dass**
das Verankerungsteil (30) derart ausgebildet ist, dass zumindest eine Querschnittsfläche eine im Wesentlichen ovale, insbesondere elliptische, Flächenbegrenzung aufweist.

11. Künstlicher Zahnersatz nach Anspruch 10
**dadurch gekennzeichnet, dass**
der Aufbauteilaufnahmebereich (36) ein Langloch umfasst, das vorzugsweise entlang der Symmetrieachse einer/der ovalen, insbesondere elliptischen, Flächenbegrenzung der Verankerungsteils (30) ausgerichtet ist.

## Claims

1. An abutment (20) for an artificial dental prosthesis, comprising:
- an upper abutment (21) arranged along a longitudinal axis (7), and
- a lower abutment (23) having such a profile for the positive-locking insertion of the abutment (20) into an abutment receiving area (26) of an anchoring part (30) that a torque applied onto the abutment (20) is transferable to the anchoring part (30),
wherein the upper abutment (21) comprises a tool fitting (40) for the positive-locking reception of a tool, wherein the abutment (20) at least in sections is configured to be trimmable as a glass-fiber-reinforced and/or carbon-fiber-reinforced plastic, wherein the fibers are oriented in parallel to the longitudinal axis (7),
**characterized in that**
a predetermined breaking point (45) delimits the torque transmission between the upper abutment (21) and the lower abutment (23) and/or between the tool fitting (40) and the lower abutment (23).

2. The abutment (20) according to claim 1,
**characterized in that**
the tool fitting (40) comprises a protrusion resting upon the upper abutment (21) along the longitudinal axis (7,) in particular on the side facing away from the lower abutment (23).

3. The abutment (20) according to anyone of the preceding claims,
**characterized by**
at least one notch for providing a/the predetermined breaking point (45).

4. The abutment (20) according to anyone of the preceding claims,
**characterized in that**
the profile of the abutment (20) comprises a multi-edge profile and/or a multiple circular shape.

5. The abutment (20) according to anyone of the preceding claims,
**characterized in that**
the tool fitting (40) comprises a multi-edge profile and/or a multiple circular shape and/or a fitting for a multi-edge profile and/or a fitting for a multiple circular shape.

6. The abutment (20) according to anyone of the preceding claims,
**characterized in that**
the upper abutment (21) is configured to be widened and/or protruding outward with respect to the lower abutment (23) for forming a contact surface (22b), wherein the contact surface preferably extends substantially perpendicular to the longitudinal axis (7).

7. An artificial dental prosthesis having an implant (10) for receiving a dental crown (1) wherein the implant (10) has an abutments (20) according to anyone of the preceding claims and an anchoring part (30), wherein the anchoring part (30) has an abutment receiving area (36) for receiving the abutment (20) and is at least in part formed of a first material, wherein the first material preferably belongs to the material group of technical ceramics.

8. The artificial dental prosthesis according to claim 7,
**characterized in that**
the anchoring part (30) has a frustoconical portion (34) with a concave envelope surface for receiving a part of a dental crown (1).

9. The artificial dental prosthesis according to claim 7 or 8,
**characterized in that**
the anchoring part (30) has at least one threaded portion (31a, 31b) for screwing the anchoring part (30) into a bone (2).

10. The artificial dental prosthesis according to claim 9,
**characterized in that**
the anchoring part (30) is configured such that at least one cross-sectional surface has a substantially oval, in particular elliptical surface delimitation.

11. The artificial dental prosthesis according to claim 10,
**characterized in that**
the abutment receiving area (36) comprises an oblong hole which is oriented preferably along the axis of symmetry of a/the oval, in particular elliptical surface

## Revendications

1. Pilier (20) pour une prothèse dentaire artificielle, comprenant :
- une partie supérieure de pilier (21) disposée le long d'un axe longitudinal (7) et
- une partie inférieure de pilier (23) qui présente, pour la mise en place par complémentarité de forme du pilier (20) dans une zone de logement de pilier (26) d'une partie d'ancrage (30), un profil tel qu'un couple appliqué au pilier (20) soit transmissible à la partie d'ancrage (30),
sachant que la partie supérieure de pilier (21) comprend un logement d'outil (40) pour le logement par complémentarité de forme d'un outil,
sachant que le pilier (20) est constitué comme matière synthétique renforcée par fibres de verre et/ou renforcée par fibres de carbone polissable au moins par sections, sachant que les fibres sont orientées parallèlement à l'axe longitudinal (7),
**caractérisé en ce que**
un point de rupture (45) limite la transmission de couple entre la partie supérieure de pilier (21) et la partie inférieure de pilier (23) et/ou entre le logement d'outil (40) et la partie inférieure de pilier (23).

2. Pilier (20) selon la revendication 1,
**caractérisé en ce que**
le logement d'outil (40) comprend un prolongement qui repose sur la partie supérieure de pilier (21) le long de l'axe longitudinal (7), en particulier du côté opposé à la partie inférieure de pilier (23).

3. Pilier (20) selon l'une des revendications précédentes,
**caractérisé par**
au moins une encoche pour la mise à disposition d'un/du point de rupture (45).

4. Pilier (20) selon l'une des revendications précédentes,
**caractérisé en ce que**
le profil du pilier (20) comprend un profil polygonal et/ou une forme circulaire multilobe.

5. Pilier (20) selon l'une des revendications précédentes,
**caractérisé en ce que**
le logement d'outil (40) comprend un profil polygonal et/ou une forme circulaire multilobe et/ou un logement pour un profil polygonal et/ou un logement pour une forme circulaire multilobe.

6. Pilier (20) selon l'une des revendications précédentes,
**caractérisé en ce que**
la partie supérieure de pilier (21) est constituée de manière élargie par rapport à la partie inférieure de pilier (23) et/ou en saillie vers l'extérieur pour constituer une surface de contact (22b), sachant que la surface de contact s'étend de préférence de manière sensiblement perpendiculaire à l'axe longitudinal (7).

7. Prothèse dentaire artificielle présentant un implant (10) pour le logement d'une couronne dentale (1), sachant que l'implant (10) présente un pilier (20) selon l'une des revendications précédentes et une partie d'ancrage (30), sachant que la partie d'ancrage (30) présente une zone de logement de pilier (36) pour le logement du pilier (20) et est constituée au moins par sections d'un premier matériau, sachant que le premier matériau fait partie de préférence du groupe de matériaux de la céramique technique.

8. Prothèse dentaire artificielle selon la revendication 7
**caractérisée en ce que**
la partie d'ancrage (30) présente une section tronconique (34) avec une virole concave pour le logement d'une partie d'une couronne dentale (1).

9. Prothèse dentaire artificielle selon la revendication 7 ou 8,
**caractérisée en ce que**
la partie d'ancrage (30) comprend au moins un section filetée (31a, 31b) pour visser la partie d'ancrage (30) dans un os (2).

10. Prothèse dentaire artificielle selon la revendication 9,
**caractérisée en ce que**
la partie d'ancrage (30) est constituée de telle manière qu'au moins une section transversale présente une limitation de surface sensiblement ovale, en particulier elliptique.

11. Prothèse dentaire artificielle selon la revendication 10,
**caractérisée en ce que**
la zone de logement de pilier (36) comprend un trou oblong qui est orienté de préférence le long de l'axe de symétrie d'une/de la limitation de surface ovale, en particulier elliptique, de la partie d'ancrage (30).
